# EUROPEAN PATENT APPLICATION

(11) **EP 2 036 562 A1**
(43) Date of publication of application: **18.03.2009**
(21) Application number: 08252607.0
(22) Date of filing: 31.07.2008
(51) Int. Cl.: A61K 31/522, A61P 31/04

(54) **A1 adenosine receptor antagonist for preventing and treating tissue injury and sepsis associated with yersinia pestis infection**

(30) Priority: 15.08.2007 US 955950
(71) Applicant: Endacea, Inc., Research Triangle Park, NC 27709-2076 (US)
(72) Inventor: Wilson, Constance N., North Carolina 27709-2076 (US)
(74) Representative: Duckworth, Timothy John

(57) **Abstract**

Methods for treating and preventing tissue injury and sepsis associated with a *Yersinia pestis* infection, particularly pneumonic plague, are provided. The methods of the invention comprise administering to a subject a therapeutically effective amount of an A₁ adenosine receptor antagonist alone or in combination with at least one additional therapeutic agent, including an antibiotic agent. The present methods find use in biodefense as a means of preventing and treating tissue injury and sepsis associated with *Y. pestis* infection, particularly pneumonic plague, in the event of a bioterrorist attack with this deadly bacterium.

## Description

### CROSS REFERENCE TO RELATED APPLICATIONS

This application claims the benefit of U.S. Provisional Application Ser. No. 60/955,950, filed August 15, 2007, which is incorporated herein by reference in its entirety.

### FEDERALLY SPONSORED RESEARCH OR DEVELOPMENT

This invention was made with United States Government support under grant number 1 R43 AI069636-01 awarded by the National Institutes of Health/National Institute of Allergy and Infectious Diseases. The government has certain rights in the invention.

### FIELD OF THE INVENTION

The present invention relates to methods for the prevention and treatment of tissue injury and sepsis associated with *Yersinia pestis* infection, particularly pneumonic plague.

### BACKGROUND OF THE INVENTION

*Yersinia pestis* is a widespread and rapidly fatal Gram-negative bacterium classified as a Category A biological agent by the Centers for Disease Control and Prevention (CDC). *Y. pestis* is the causative agent of the plague, one of the most feared contagious epizootic diseases in history. See, for example, Perry and Fetherston (1997) Clin. Microbiol. Rev. 10:35-66; Cantor (2001) In the Wake of the Plague New York: Simon & Schuster; and Inglesby et al. (2000) JAMA 283:2281-229. Three clinical forms of plague exist in humans: bubonic, septicemic, and pneumonic plague. Bubonic plague usually results from the bite of an infected rodent flea and is characterized by the sudden onset of fever, headache, chills, nausea, vomiting, abdominal pain, and weakness within two to eight days of inoculation. See Josko (2004) Clin. Lab. Sci. 17:25-29. A tender swelling of the regional lymph nodes that drain the affected skin results within a few days of infection. Kool (2005) Healthcare Epidemiology 40:1166-1172. This form of plague is not transmitted from person to person but may result in secondary pneumonic plague if the bacteria reach the lungs. *Id*. Secondary pneumonic plague occurs in less than 5% of patients that are promptly treated for bubonic plague, with that number rising significantly in the absence of treatment. *Id*. Septicemic plague occurs when the *Y. pestis* bacteria multiply in the blood stream, usually as a complication of untreated bubonic or pneumonic plague. See, for example the National Institute of Allergy and Infectious Diseases website at niaid.nih.gov/factsheets/plague.htm.

Pneumonic plague is the most serious form of *Y. pestis* infection and occurs when the bacteria infect the lungs and cause pneumonia. Primary pneumonic plague results from direct inhalation of the *Y. pestis* bacteria, such as by airborne transmission from an infected person to an uninfected individual or by intentional release of aerosolized bacteria (e.g., a bioterrorist attack). Kool, *supra.* Secondary pneumonic plague, as noted above, results from the spread of *Y*. *pestis* via the bloodstream to the lungs in patients afflicted with bubonic plague. Pneumonic plague has an incubation period of approximately 1-6 days and is characterized by, for example, the sudden onset of severe headache, chills, malaise, and increased respiratory and heart rates. *Id.* These symptoms rapidly progress to pneumonia and may ultimately lead to respiratory failure if left untreated. Josko, *supra.* Both primary and secondary forms of pneumonic plague can be spread from person to person via inhalation of contaminated aerosol droplets. *Id.*

Today, the first-line therapy for *Y. pestis* infection is treatment with antibiotics. Prior to the advent of antibiotic therapies, the mortality rate of pneumonic plague was nearly 100%. Kool, *supra.* Appropriate antibiotics, if administered in a timely fashion (i.e., within approximately 20 hours of the onset of the disease) reduce mortality rates, but the fatality rates for pneumonic plague still remain high, particularly following a potential bioterrorist event in which numerous individuals would be exposed to potentially antibiotic-resistant *Y. pestis* bacteria. Furthermore, antibiotic treatment alone is insufficient for some patients suffering from pneumonic plague, who may also require circulatory support, ventilatory support, or renal support.

According to military and other government agencies, at least 10 countries have the capability to produce and disseminate biological weapons, and it is unknown how many terrorist groups and rogue nations share this capability. As recognized and appreciated by the CDC and Homeland Security agencies, *Y. pestis* is a logical candidate for a potential bioterrorist weapon and poses a particularly dangerous threat because of: 1) its natural occurrence on every continent, 2) the ease of its dissemination from wild and domesticated animal reservoirs, as well as man-made devices, 3) a striking dearth of current experience with its clinical presentation coupled possibly with physician complacency in this era of readily available antibiotics, 4) the ability to mass produce the bacteria, and 5) the ease by which genetically modified, antibiotic-resistant strains can be produced. See, for example, Finegold et al. (1968) Am. J. Path. 53:99-114; Walker (1968) Curr. Top. Micro. Immun. 41:23-42; Walker (1968) J. Infect. Dis. 118:188-96; Beebe and Pirsch (1958) Appl. Microbiol. 6:127-138; Williams et al. (1994) J. Wildlife Dis. 30:581-585; Watson et al. (2001) Veter. Path. 38:165-172; and Green et al. (1999) FEMS Immun. Med. Micro. 23:107-113

Increasing the potential for *Y. pestis* to be used as a bioweapon is the development of multi-drug resistant strains of the bacterium. In the event of a bioterrorist attack with *Y*. *pestis,* antibiotics also may not be available in sufficient quantities or be able to be administered in a timely manner for the prevention of death following inhalation of sufficient amounts of *Y. pestis.* The epidemiology of primary pneumonic plague following a bioterrorist attack would differ significantly from that of a naturally occurring infection. Symptoms would appear 1 to 3 days following exposure and would initially resemble other severe respiratory infections. The disease would rapidly progresses to gram-negative septicemia with respiratory failure, circulatory collapse, and disseminated intravascular coagulation. See Gradon (2002) Curr. Infect. Dis. Rep. 4:244-248. With prompt treatment within 24 hours of the onset of symptoms, fatality rates would be 50-60%. See, for example, Inglesby et al. (2000) JAMA 283:2281-2290 and Lane et al. (2001) Nat. Med. 7:1271-1273. If treatment were delayed for more than 24 hours, the fatality rate of pneumonic plague would be nearly 100%. Moreover, in a government exercise designed to simulate an aerosolized *Y. pestis* attack, it was evident that the disease would spread rapidly and that hospitals would be quickly overwhelmed and unable to care for infected patients. Inglesby et al. (2001) Clin. Infect. Dis. 32:436-445. Because of the limited supply of first-line antibiotics in the National Stockpile, it is also highly unlikely that all victims could be treated in time to prevent mass casualties. Therefore, there is a critical need to develop novel therapeutic strategies to expand the window of opportunity for treating patients following a bioterrorist attack with this deadly bacterium and for preventing the development of pneumonic plague in those exposed but not yet afflicted with the disease.

Sepsis is often associated with a Gram-negative bacterial infection, such as *Y. pestis* infection. The term "sepsis" as used herein is inclusive of sepsis, septicemia (bacteremia/endotoxemia), severe sepsis, septic shock, and related conditions, as well as the clinical symptoms and complications associated with each of these conditions. Most of the damage associated with Gram-negative sepsis comes not from the invasion of bacteria *per se* but from the endotoxin present in the cell wall of the bacteria. Lipopolysaccharide ("LPS" or "endotoxin"; used interchangeably herein), is a glycolipid-rich moiety that forms the major constituent of the outer wall of gram-negative bacteria. Endotoxin is a toxin released during the normal growth, death, and lysis of Gram-negative bacteria. Following its release into the blood stream from a site of infection, such as the lung, abdomen, or urinary tract, endotoxin acts on a number of different cell types, and induces a complex cascade of cellular, mediator and cytokine-related events. This inflammatory cascade results in organ (e.g., lung and kidney) damage, shock and death in patients with Gram-negative septicemia (endotoxemia). Based on the current understanding of how endotoxin induces this complex cascade of events, specific therapies developed in the past or currently in development attempt to target specific events in this cascade.

Lipopolysaccharide is a recognized virulence factor for *Y. pestis.* See Bruneteau and Minka (2003) Biochimie 85:145-152; Dalla et al. (1985) Eur. J. Biochem. 151:399-404; and Minka and Bruneteau (1998) Can. J. Microbiol. 44:477-481. LPS is released during the normal growth, death, and lysis of *Y. pestis* bacteria and represents a potentially fatal insult to host tissues associated with *Y. pestis* infection. In the lung, the presence of LPS causes structural changes resulting in disruption of the blood-air barrier, interstitial and alveolar edema, inflammatory cellular infiltration, microvascular congestion and eventually frank alveolar hemorrhage. These pathological changes of acute lung injury (ALI) produced by LPS are similar to those caused by *Y. pestis* in humans and animals. See Inglesby et al. (2000) JAMA 283:2281-2290; Watson et al. (2001) Veter. Path. 38:165-172; Finegold (1969) Am. J. Path. 54:167-185; Walker (1968) Curr. Top. Micro. Immun. 41:23-42; Walker (1968) J. Infect. Dis. 118:188-96; Walker (1972) SE As.J.Trop.Med.Pub.HI. 3:221-224; Butler and Moller (1977) Infect. Immun. 18:400-404; Butler et al. (1976) J. Infect. Dis. 133:493-499; and Revell and Miller (2001) FEMS Micro. Lett. 205:159-164.

Endotoxin also induces tissue injury or dysfunction in a number of organs or tissues including, but not limited to, the lung, heart, eye, intestines, peritoneum, liver, stomach, ear, blood vessels, nerves, skin, brain and spinal cord, sinus and nose, kidney, testis and epididymis, ovary, fallopian tube, prostate, placenta, bronchi, and gum tissue. See, e.g., Lew et al. (1997) Am. J. Physiol. 272:H2989-H2993; Goddard et al. (1996) Am. J. Physiol. 270:H 1446-H 1452; Behar-Cohen et al. (1997) Exp. Eye Res. 65:533-545; Unno et al. Gastroenterology 113:1246-1257; Vos et al. (1997) Gastroenterology 113:1323-1333; Abel-Aziz et al. (1997) J. Clin. Biochem. Nutr. 22:19-29; Sakagami et al. (1997) Infection and Immunity 65:3310-3316; Sugiura et al. (1997) Acta Otolaryngol. 531:21-33; McKenna (1990) J. Clin. Invest. 86:160-168. Moreover, endotoxin enhances organ injury associated with certain toxins, alcohol, parasitic infections, and ischemia and reperfusion. Vogt et al. (1995) Lab. Invest. 72:474-483; Arai et al. (1989) Jap. J. Gastroenterology 86:1089-1095; Abel-Aziz et al. (1997) J. Clin. Biochem. Nutr. 22:19-29; and Liu et al. (1994) Circ. Shock 43:9-17.

Previously it was reported that A₁ adenosine receptor antagonists block LPS-induced ALI in animals. Neely et al. (1997) Am. J. Physiol 272:L353-L361. Moreover, it has been reported that LPS binds to and activates A₁ adenosine receptors on human pulmonary artery endothelial cells (PAECs) to induce the release of thromboxane (TXA2), and interleukin (IL)-6, substances which are cytotoxic to endothelial cells. Wilson and Batra (2002) J. Endotoxin. Res. 8:263-271.

Although *Y. pestis* infection is rarely seen today by medical professionals in the U.S. (10-20 cases/year in the U.S.; 1000-3000 cases worldwide/year, according to the CDC), *Y. pestis* infection remains a significant concern as a potential biological weapon. Accordingly, there is a need in the art to develop novel prophylactic and post-infection regimens for the treatment of *Y. pestis* infections (e.g., bubonic, septicemic, and pneumonic plague), as well as education of medical practitioners to permit the rapid identification of the signs and symptoms of *Y. pestis* infection, particularly pneumonic plague. Moreover, methods for treating primary and secondary pneumonic plague are needed, specifically to combat the threat of a bioterrorist event involving an aerosolized, potentially antibiotic-resistant version of *Y. pestis.* Moreover, because the mortality rate for *Y. pestis* infections is high (50-60%), even with prompt treatment within 24 hours of onset of symptoms, adjunctive therapies to antibiotics that block *Y*. *pestis* LPS-induced ALI and other tissue injuries, septicemia, shock, and death are needed. The prophylactic use of novel therapies that block *Y. pestis* LPS-induced tissue injury and sepsis, may provide a therapeutic window to protect the subject from tissue injury, septicemia, and death exposed to *Y. pestis* but without signs and symptoms of the infection until the appropriate antibiotics and other supportive care can be administered.

### BRIEF SUMMARY OF THE INVENTION

Methods for preventing and treating tissue injury and sepsis associated with *Y. pestis* infection (i.e., the causative agent of bubonic plague, septicemic plague, and pneumonic plague) are provided. The methods of the invention comprise administering to a subject a therapeutically effective amount of an A₁ adenosine receptor antagonist. The A₁ adenosine receptor antagonist may be administered alone or in combination with an at least one additional therapeutic agent (e.g., an antibiotic agent) to promote a desired prophylactic or therapeutic response. When the A₁ adenosine receptor antagonist is administered in combination with at least one therapeutic agent, the A₁ adenosine receptor antagonist and the additional therapeutic agent(s) may be administered simultaneously or sequentially. The methods for preventing or treating tissue injury and sepsis associated with *Y. pestis* infection disclosed herein further comprise administering a pharmaceutical composition to a subject, wherein the pharmaceutical composition comprises an A₁ adenosine receptor antagonist, a pharmaceutically acceptable carrier, and optionally at least one additional therapeutic agent, such as an antibiotic agent. Methods of the invention find use in treating subjects currently suffering from *Y. pestis* infection, particularly pneumonic plague, and patients at risk for exposure to and infection by *Y. pestis,* such as, for example individuals exposed to *Y. pestis* during a bioterrorist attack.

### BRIEF DESCRIPTION OF THE DRAWINGS

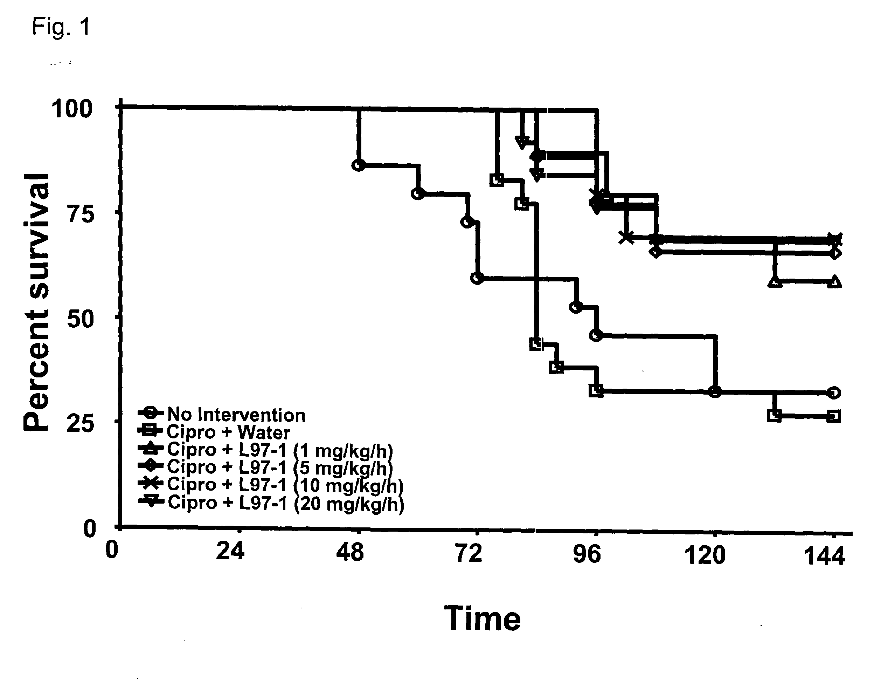

Figure 1 provides the survival rates for rats post-intratracheal infection with *Y. pestis* and treated at 72 hours post-infection with no intervention, cipro + water, cipro + L-97-1 (1 mg/kg/h), cipro + L-97-1 (5 mg/kg/h), cipro + L-97-1 (10 mg/kg/h) or cipro + L-97-1 (20 mg/kg/h). Experimental details are provided in Example 2.

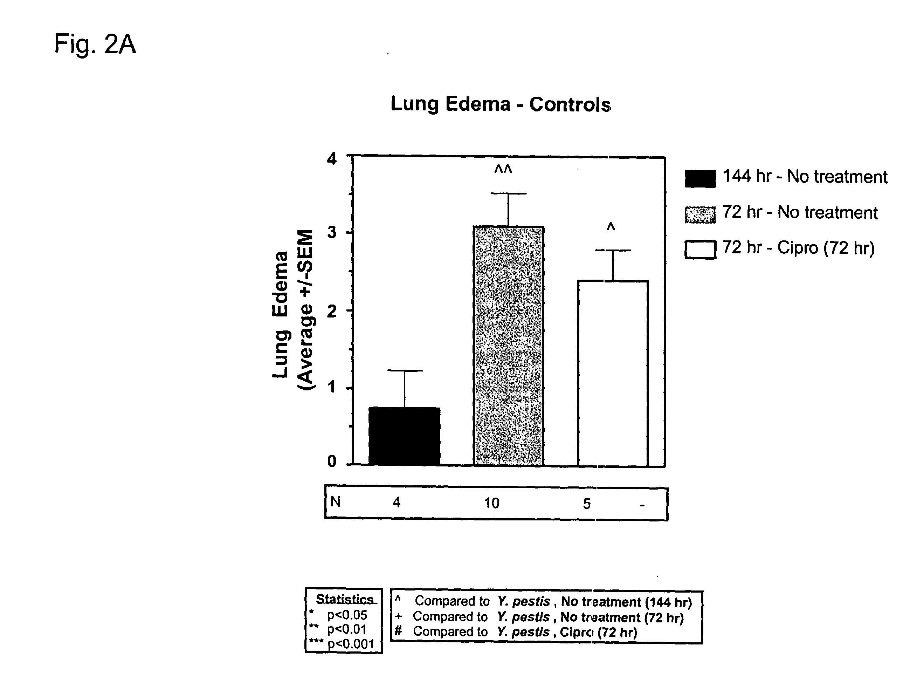

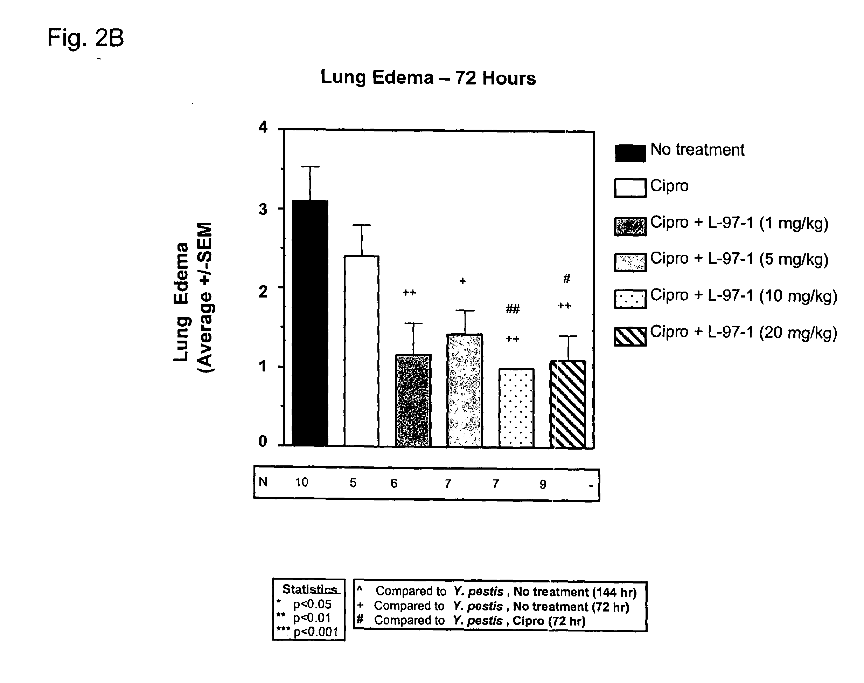

Figure 2A provides the control lung edema values for rats post-intratracheal infection with *Y*. *pestis* and left untreated at 72 hours and 144 hours or treated with cipro at 72 hours post-infection. Figure 2B provides the lung edema values for rats post-intratracheal infection with *Y. pestis* at 72 hours post-infection and left untreated or treated with cipro alone, cipro + L-97-1 (1 mg/kg/h), cipro + L-97-1 (5 mg/kg/h), cipro + L-97-1 (10 mg/kg/h), or cipro + L-97-1 (20 mg/kg/h). Experimental details are provided in Example 2.

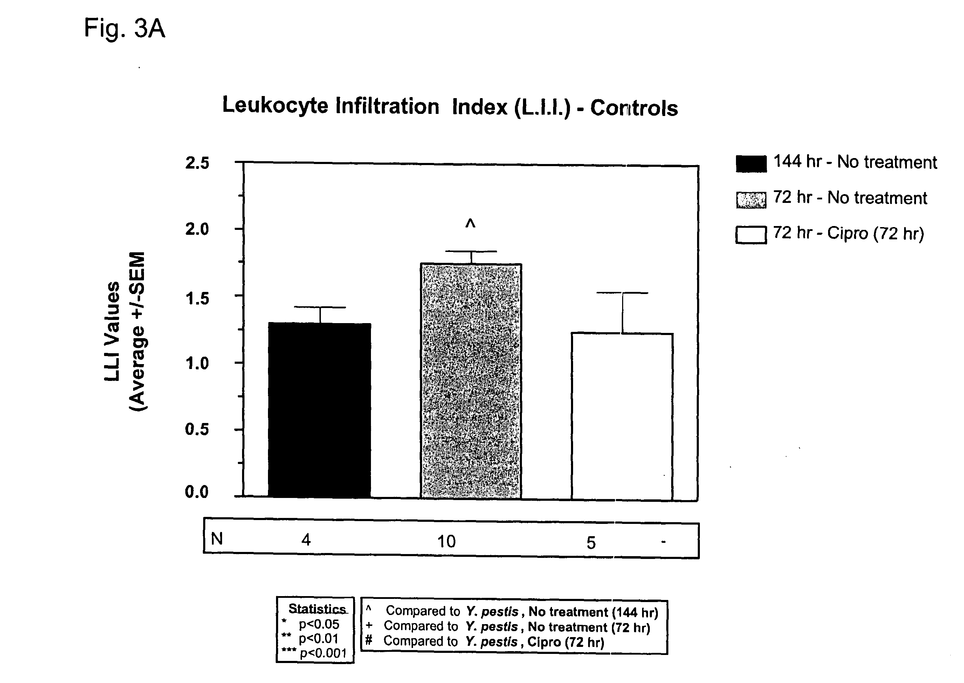

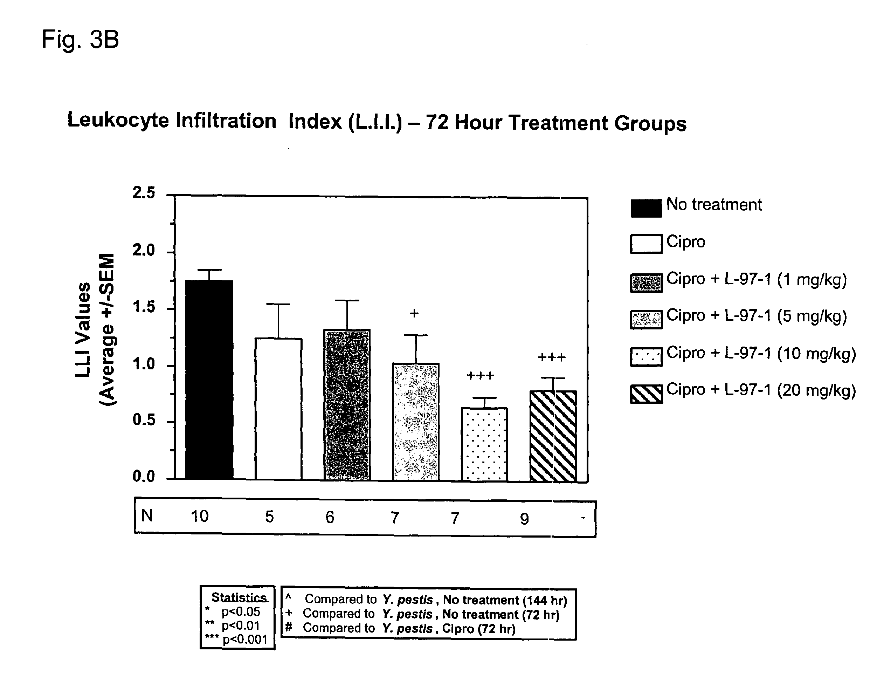

Figure 3A provides the control leukocyte infiltration index (L.I.I.) values for rats post-intratracheal infection with *Y. pestis* and left untreated at 72 hours and 144 hours or treated with cipro at 72 hours post-infection. Figure 3B provides the L.I.I. values for rats post-intratracheal infection with *Y. pestis* at 72 hours post-infection and left untreated or treated with cipro alone, cipro + L-97-1 (1 mg/kg/h), cipro + L-97-1 (5 mg/kg/h), cipro + L-97-1 (10 mg/kg/h), or cipro + L-97-1 (20 mg/kg/h). Experimental details are provided in Example 2.

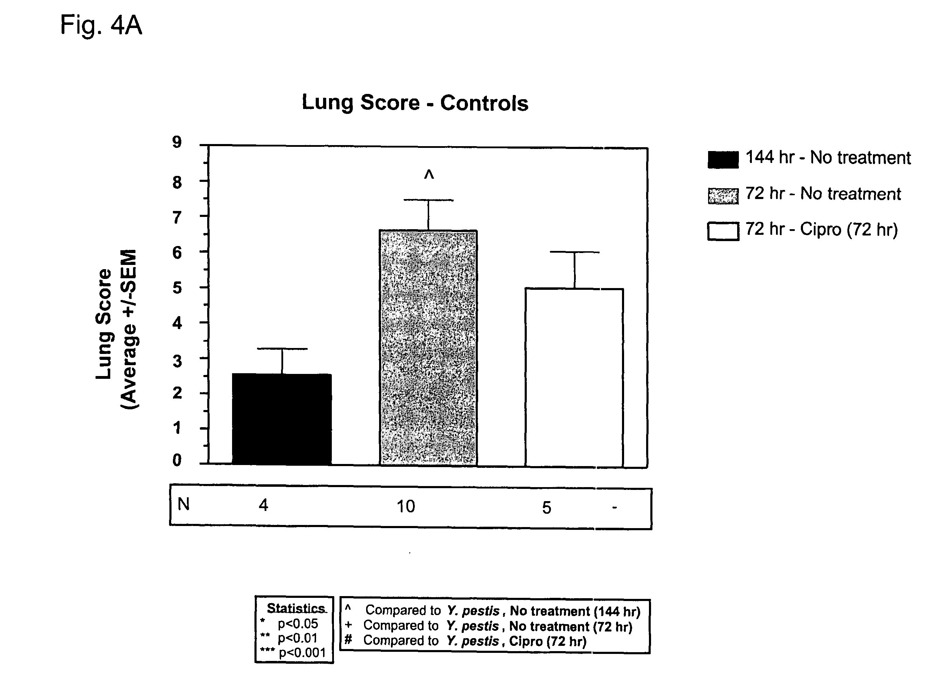

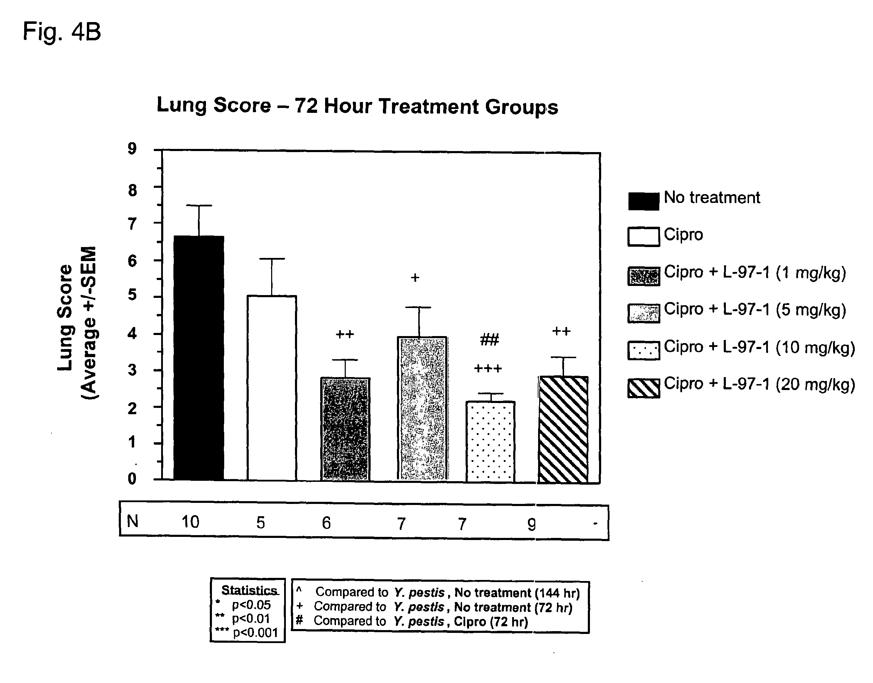

Figure 4A provides the control lung scores for rats post-intratracheal infection with *Y. pestis* and left untreated at 72 hours and 144 hours or treated with cipro at 72 hours post-infection. Figure 4B provides the lung scores for rats post-intratracheal infection with *Y. pestis* at 72 hours post-infection and left untreated or treated with cipro alone, cipro + L-97-1 (1 mg/kg/h), cipro + L-97-1 (5 mg/kg/h), cipro + L-97-1 (10 mg/kg/h), or cipro + L-97-1 (20 mg/kg/h). Experimental details are provided in Example 2.

### DETAILED DESCRIPTION OF THE INVENTION

The present invention is directed to methods for preventing or treating tissue injury and sepsis associated with *Y. pestis* infection, more particularly pneumonic plague. The methods comprise administering to a subject a therapeutically effective amount of an A₁ adenosine receptor antagonist alone or in combination with at least one additional therapeutic agent such as, for example, an antibiotic agent. A₁ adenosine receptor antagonists are known in the art and include, for example, those compounds described in U.S. Pat. Nos. 5,786,360, 6,489,332, 7,202, 252 B2, 7,247,639 B2, and in co-pending U.S. Application No.10/560,853, entitled "A₁ Adenosine Receptor Antagonists," filed June 7, 2004, all of which are herein incorporated by reference in their entirety. In some embodiments, the A₁ adenosine receptor antagonist comprises a compound of formula (I): 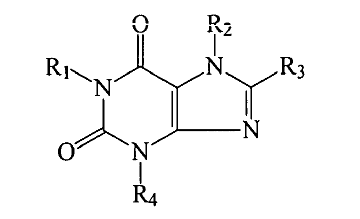 wherein R₁ is selected from the group consisting of C₁-C₈ alkyl;

R₂ is of the formula: 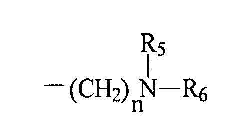 wherein n is an integer ranging from 1 to 8; R₅ is H or CH₃(CH₂)ₚ, wherein p is an integer ranging from 1 to 7; and R₆ is H; (CH₂)ₘH; or (CH₂)ₘOH, wherein m is an integer ranging from 1 to 8;

R₃ is:

-(CH₂)_{q}C₆H₄-R₇

wherein q is an integer ranging from 1 to 8; wherein R₇ is selected from the group consisting of H, OH, NH₂, R₉COOH, wherein R₉ is an alkylene or alkenylene group having 1 to 8 carbon atoms, and (CH₂)ₜOH, wherein t is an integer ranging from 1 to 8; -and

R₄ is of the formula: 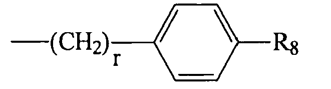 wherein R₈ is selected from the group consisting of H, NH₂, OH, (CH₂)_{f}NH₂ wherein f is an integer ranging from 1 to 8, (CH₂)ₛOH, wherein s is an integer ranging from 1 to 8, and R₁₀COOH, wherein R₁₀ is an alkylene or alkenylene group having 1 to 8 carbon atoms; and r is an integer ranging from 1 to 8. Methods for synthesizing the A₁ adenosine receptor antagonists of the invention are known in the art and are described in, for example, U.S. Pat. Nos. 5,786,360, 6,489,332, 7,202, 252 B2, and 7,247,639 B2.

In a particular aspect of the invention, the A₁ adenosine receptor antagonist is 3-[2-(4-aminophenyl)-ethyl]-8-benzyl-7-{2-[ethyl-(2-hydroxyethyl)-amino]-ethyl}-1-propyl-3,7-dihydropurine-2,6-dione, designated L-97-1, and comprises the compound of formula (I), wherein:
R₁ is C₃ alkyl;
R₂ is: 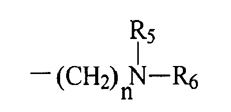 wherein n is 2; R₅ is CH₃(CH₂)ₚ, wherein p is 1; and R₆ is (CH₂)ₘOH, wherein m is 2;
R₃ is:

   -(CH₂)_{q}C₆H₄-R₇

   wherein q is 1; wherein R₇ is H; -and
R₄ is of the formula: 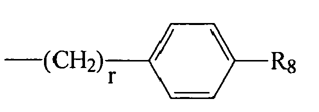 wherein R₈ is NH₂; and r is 2. Additional data regarding the pharmacological and pharmacokinetic properties of L-97-1 are disclosed in U.S. Patent Application No. 11/412,754 entitled "Methods and Pharmaceutical Compositions for Treating Sepsis," filed April 27, 2006, the contents of which are herein incorporated by reference in their entirety.

The methods disclosed herein further comprise administering at least one additional therapeutic agent in combination with the A₁ adenosine receptor antagonists of the invention. As used herein, "therapeutic agent" refers to any compound, the co-administration of which with an A₁ adenosine receptor antagonist treats, prevents, or limits the progression of tissue injury and sepsis associated with *Y*. *pestis* infection, more particularly pneumonic plague. Therapeutic agents include but are not limited to antibiotic agents. Other agents include agents that support the heart and blood pressure, including dopamine, dobutamine, epinephrine, and norepinephrine, support the kidney, including diuretics, ACE inhibitors, and mannitol, and agents used to treat gram-negative septicemia, including steroids, insulin, and recombinant activated protein C (APC; drotrecogin alpha; Xigris^{®}. In particular, methods of preventing or treating tissue injury and sepsis associated with *Y. pestis* infection, more particularly primary or secondary pneumonic plague, comprising administering an A₁ adenosine receptor antagonist in combination with an antibiotic agent are contemplated by the invention. Furthermore, methods of treating or preventing tissue injury and sepsis associated with *Y. pestis* infection (e.g., bubonic plague, septicemic plague, or pneumonic plague) comprising the administration of a pharmaceutical composition to a subject, wherein the pharmaceutical composition comprises an A₁ adenosine receptor antagonist, a pharmaceutically acceptable carrier, and optionally at least one additional therapeutic agent, particularly an antibiotic agent, are further provided.

By "antibiotic agent" is intended any substance which inhibits the growth of or kills susceptible bacteria or other microorganisms, specifically *Y. pestis.* Any antibiotic agent or combination of antibiotic agents may be used to practice the invention, such as, for example, aminoglycosides and tetracyclines. Antibiotic agents further include but are not limited to ciprofloxacin, streptomycin, chloramphenicol, tetracycline, doxycycline and gentamicin. Antibiotic agents include but are not limited to β-lactams (e.g., penicillin, ampicillin, cefuroxime, ceftazidime, and imipenem), aminoglycosides (e.g., gentamicin, amikacin, kanamycin, tobramycin, and netilmicin), carbapenems, cephalosporins (e.g., cefotaxime, moxalactam, and cefoperazone), penicillin, amoxicillin, clindamycin, carboxypenicillins (e.g. ticarcillin), ureidopenicillins (e.g., piperacillin), β-lactamase inhibitors (e.g., clavulanic acid and tazobactam), fluoroquinones (e.g., ciprofloxacin, norfloxacin, and levofloxacin), glycopeptides (e.g., vancomycin and teicoplanin), oxazolidinones (e.g., linezolid), and streptogramins (e.g., quinupristin/dalfopristin). See generally Bochud et al. (2004) Crit. Care Med 32(11) 495-512 and Harris and Thorarensen (2004) Curr. Med. Chem. 11:2213-2243, both of which are herein incorporated by reference in their entirety. Furthermore, antibiotic agents may include sulfonamides, chloramphenicol, tetracyclines, macrolides, lincosamides, rifamycins, nitromidiazoles, quinolones, trimethoprim, and lipopeptides. Powers (2004) *Clin*. *Microbiol*. *Infect*. 10 (Supp. 4):23-31 (See particularly p. 24, Table 1).

Antibiotic agents may include those from new emerging classes of antibiotics including, inhibitors of DNA methyltransferase, pyrrole tetramide DNA binders, heteroaromatic polycyclic (HARP) compounds, anti-bacterial DNA binders, benzamides, benzothiophenes, isoquinoline analogs, gyrase inhibitors, pyrido [1,2-c] pyrimidine gyrase inhibitors, benzimidazole/benzoxazole gyrase inhibitors, quinazolinedione gyrase inhibitors, PcrA inhibitors, inhibitors of RNA polymerase, RNA bacterial ribosome targets, including protein synthesis inhibitors, inhibitors of RNA-protein interactions (complexes), transcriptional/translational inhibitors, paromomycin, elongation inhibitors, translation inhibitor TAN-1057, aminoacyl- tRNA synthetase inhibitors, chuangximycin analogs, peptide deformylase (PDF) inhibitors, bacterial cell wall inhibitors, including UDP-*N*-aceytylmuramate/L-alanine ligase (Mur C) and other mur (D and I) inhibitors, as well as phosphor-N-acetylmuramyl-pentapeptide translocase (Mra Y), including muraymycin C1, muraymycin A1, mureidomycin A, liposidomycin C, RU75411, penicillin binding protein (PBP) inhibitors, inhibitors of bacterial cell membranes, including inhibitors of lipid A biosynthesis, metalloenzyme inhibitors, hydroxamic acid inhibitors, including BB-78484, BB-78485, inhibitors of 3-deoxy-D-manno-2-octulosonate -8-phosphate synthetase (KDOP), mutulin derivatives, althiomycin and analogs of althiomycin, naphthyridine agents, pyrimidine-pyridine analogs, piperidine agents, tetrahydroquinoline analogs, mannopeptimycin, AC-98-5, AC-98-6446, phosphoryl transfer system (PTS) inhibitors, AI-2 signaling pathway inhibitors, dehydroquinate synthetase (DHQS) inhibitors, Ar-358, shikimate kinase inhibitors, chorismate synthase (CS) inhibitors, PTX110130, PTX008313, nicotinamide adenine dinucleotide (NAD) synthetase inhibitors, fatty acid biosythesis inhibitors, thiolactomycin, triclosan, isoniazid, cerulenin, phosphopantetheine adenylyltransferase (PPAT) inhibitors, PTX-042695, PTX-031553, PTX-007063, PTX-008134, Fab inhibitors, β-ketoacyl-acyl carrier protein (ACP) synthase III (FABH) inhibitors, thiolactomycin analogs, enoyl-ACP reductase (FabI or FabK) inhibitors, SB-627696, SB-663042, and SB-633857. See generally Harris and Thorarensen, *supra*.
In a particular embodiment of the invention, the antibiotic agent is a broad-spectrum antibiotic. Antibiotic agents of particular interest include but are not limited to gentamicin, tobramycin, clinamycin, cefotaxime, amikacin, imipenem, netilycin, ceftazidime, cefuroxamine, metronidazole, cefazolin, cefoperazone, ceftriaxone, mezlocilin, ampicillin, amoxiclav, piperacillin, tazobactam, and ciprofloxacin. See, for example, Bochud *et al*., *supra* (particularly Tables 1 and 2).

One of skill in the art will appreciate that the choice of antibiotic agent will be influenced by the susceptibility patterns of the specific strain of *Y*. *pestis* involved, particularly if the *Y. pestis* strain has been genetically modified to be resistant to certain antibiotics, and the patient's past medical history with a particular antibiotic (e.g., allergies, etc.). The use of multiple antibiotics (e.g., 2, 3, 4, 5, or more antibiotics) in combination with an A₁ adenosine receptor antagonist of the invention is also encompassed herein.

The methods of the invention are useful in treating and preventing tissue injury and sepsis associated with *Y. pestis* infection, particularly primary or secondary pneumonic plague, more particularly primary pneumonic plague. As discussed above, *Y. pestis* is the causative agent of bubonic plague, septicemic plague, and pneumonic plague. Pneumonic plague may result as a consequence of untreated bubonic or septicemic plague (i.e., secondary pneumonic plague) or by direct inhalation of aerosolized *Y. pestis* (e.g., primary pneumonic plague) such as in a bioterrorist attack. Although the treatment and prevention of all types of *Y. pestis* infections and conditions associated therewith are contemplated by the present invention, prevention and treatment of primary pneumonic plague is of particular interest.

Pneumonic plague (both the primary and secondary forms) is a potentially fatal disease caused by *Y. pestis* that is characterized by involvement of the lungs. Signs and symptoms of pneumonic plague include but are not limited to fever, chills, malaise, headache, fever, rapid or difficult breathing, increased heart rate, pneumonia (particularly rapidly progressing pneumonia), blood in the sputum, bright red sputum, foamy red sputum, and rapid shock. *Y. pestis,* particularly the endotoxin associated with this Gram-negative bacterium, can lead to rapid endotoxemia/septicemia and damage to tissues and organs, particularly in the lungs resulting in acute lung injury ("ALI"). Moreover, infiltration of the *Y. pestis* bacteria in the lung may result in ALI. Multi-organ failure may also result from the invasion of *Y*. *pestis* or the endotoxemia/septicemia associated with *Y. pestis* infection. If left untreated, and even in a significant percentage of treated patients, pneumonic plague is fatal. One of skill in the art will appreciate that diagnostic tests for pneumonic plague (or *Y. pestis* infections generally) include but are not limited to blood tests, radiological tests (e.g., to observe potential alveolar infiltrates, particularly in the lower lung lobes and nodular or patchy regions of the lungs), microbiological examination of respiratory secretions or lung tissue, sputum tests, and lymph tests for the presence of *Y. pestis.* Methods and laboratory tests for assessing the clinical symptoms (and the improvement or worsening thereof) of *Y. pestis* infection, particularly pneumonic plaque, are known in the art. Moreover, the CDC has published guidelines for the identification of *Y*. *pestis* infection. See, for example, Josko, *supra*.

The term "*Y*. *pestis* infection" as used herein is inclusive of all conditions caused by infection of a subject with *Y. pestis,* including but not limited to bubonic plague, septicemic plague, pneumonic plague, and related conditions, as well as the clinical symptoms and complications associated with each of these conditions (e.g., septicemia, acute lung injury (ALI), tissue and organ damage, multiple organ failure, shock, etc.). The methods of the invention find use, for example, in preventing, limiting, or treating *Y. pestis* infection (e.g. bubonic plague, septicemic plague, and pneumonic plague), preventing or limiting tissue or organ injury resulting from *Y. pestis* infection, particularly endotoxin-induced organ (e.g., lung) injury, shock, and death, and generally improving the outcome of patients infected with *Y. pestis.* The methods of the invention find particular use in preventing and treating primary and secondary pneumonic plague, more particularly primary pneumonic plague, as described herein above and in the art.

The methods of treatment of the present invention are not intended to be limited to particular subjects. A variety of subjects, particularly mammals, are contemplated. Subjects of interest include but are not limited to humans, dogs, cats, horses, pigs, cows, and rodents. In particular embodiments, the subject is a human. The subjects of the invention may be suffering from the symptoms of *Y. pestis* infection or may be at risk for contracting a *Y. pestis* infection, particularly pneumonic plague, more particularly primary pneumonic plague following a bioterrorist attack.

"Treatment" is herein defined as the administration of an A₁ adenosine receptor antagonist, alone or in combination with the administration of at least one additional therapeutic agent, to a subject, where the subject has a *Y. pestis* infection (bubonic plague, septicemic plague, or pneumonic plague, particularly primary pneumonic plague) or a symptom of a *Y. pestis* infection, where the purpose is to cure, heal, alleviate, relieve, alter, remedy, ameliorate, improve, or affect the condition or the symptoms of the *Y. pestis* infection. When a combination of therapeutic agents is used (e.g., an A₁ adenosine receptor antagonist and an antibiotic agent), "treatment" is also intended that the combination of the A₁ adenosine receptor antagonist and the second therapeutic agent is administered to the subject as part of a single pharmaceutical composition, or alternatively as part of individual pharmaceutical compositions, each comprising either the A₁ adenosine receptor antagonist or the second therapeutic agent, where the subject is infected with or displays at least one symptom of a *Y*. *pestis* infection, where the purpose is to cure, heal, alleviate, relieve, alter, remedy, ameliorate, improve, or affect the condition or at least one of the symptoms of the *Y. pestis* infection.

Methods for preventing tissue injury and sepsis associated with *Y. pestis* infection (e.g., bubonic plague, septicemic plague, or primary or secondary pneumonic plague, more particularly primary pneumonic plague) are also provided. By "preventing tissue injury and sepsis associated with *Y. pestis* infection" is intended that an A₁ adenosine receptor antagonist alone or in combination with at least one additional therapeutic agent (e.g., an antibiotic agent) is administered to a subject at risk for a *Y. pestis* infection, including but not limited to primary pneumonic plague, in order to prevent the development of tissue injury and sepsis associated with a *Y. pestis* infection. Such prophylactic or preventative methods find particular use following a bioterrorist or suspected bioterrorist attack with aerosolized *Y. pestis.*

In certain aspects of the invention, the methods comprise using a combination therapy. The term "combination therapy" is used in its broadest sense and means that a subject is treated with at least two therapeutic agents, more particularly an A₁ adenosine receptor antagonist and a second therapeutic agent (e.g., an antibiotic agent). The timing of administration of the A₁ adenosine receptor antagonist and the second therapeutic agent can be varied so long as the beneficial effects of the combination of these agents are achieved. The phrase "in combination with" refers to the administration of an A₁ adenosine receptor antagonist with another therapeutic agent either simultaneously, sequentially, or a combination thereof. Therefore, a subject undergoing a combination therapy of the invention can receive the A₁ adenosine receptor antagonist and the second therapeutic agent at the same time (i.e., simultaneously) or at different times (i.e., sequentially, in either order, on the same day or on different days), so long as the therapeutic effect of the combination of both agents is achieved in the subject undergoing therapy. Where the A₁ adenosine receptor antagonist and the second therapeutic agent are administered simultaneously, they can be administered as separate pharmaceutical compositions, each comprising either an A₁ adenosine receptor antagonist or the second therapeutic agent (e.g., an antibiotic agent), or can be administered as a single pharmaceutical composition comprising both agents.

The methods of the invention comprise administering to a subject a therapeutically effective amount of an A₁ adenosine receptor antagonist alone or in combination with at least one additional therapeutic agent. Any method for administering a composition to a subject may be used in the practice of the invention. Examples of possible routes of administration include parenteral, (e.g., intravenous (IV), intramuscular (IM), intradermal (ID), subcutaneous (SC), or infusion), oral, pulmonary (e.g., inhalation), nasal, transdermal (topical), transmucosal, intraperitoneal (IP), and rectal administration. In particular embodiments, the route of administration of the A₁ adenosine receptor antagonist and at least one antibiotic agent is by IV administration. By "therapeutically effective dose," "therapeutically effective amount," or "effective amount" is intended an amount of the A₁ adenosine receptor antagonist that, when administered alone or in combination with an amount of at least one additional therapeutic agent (e.g., an antibiotic agent), brings about a positive therapeutic response with respect to treatment of a subject for a *Y. pestis* infection such as bubonic plague, septicemic plague, or pneumonic plague, more particularly primary pneumonic plague. In certain embodiments, a therapeutically effective dose of the A₁ adenosine receptor antagonist for oral administration is in the range from about 0.1 mg/kg to about 50 mg/kg. In other embodiments, a therapeutically effective dose of the A₁ adenosine receptor antagonist for intravenous administration is in the range from about 0.001 mg/kg to about 25 mg/kg.

A "positive therapeutic response" refers to, for example, improving the condition of at least one of the symptoms of a *Y. pestis* infection, preventing the worsening of at least one *Y. pestis* infection-related symptom, or preventing or limiting the progression of the tissue injury and sepsis associated with *Y. pestis* infection. In particular aspects of the invention, the methods are directed to the treatment or prevention of pneumonic plague, particularly primary or secondary pneumonic plague, more particularly primary pneumonic plague. An improvement in at least one of the symptoms of pneumonic plague can be assessed by a physician using routine laboratory tests (e.g., blood tests, sputum analysis, lymph node analysis), assessment of physiological data (e.g., blood pressure, heart rate, respiratory rate, oxygenation, and mental status), chest radiograph and other radiological criteria, and standard physical examination of the patient. Determination of therapeutically effective amounts is well within the capability of those skilled in the art.

In the case of sepsis associated with a *Y. pestis* infection, a "positive therapeutic response" refers to, for example, improving the condition of at least one of the symptoms of sepsis, preventing the worsening of at least one sepsis-related symptom, or preventing or limiting the progression of the condition to subsequent stages in the sepsis cascade (e.g., severe sepsis, septic shock, organ damage, etc.). An improvement in at least one of the symptoms of sepsis can be assessed by a physician using routine laboratory tests, assessment of physiological data, (e.g., blood pressure, heart rate, respiratory rate, mental status, urine output, oxygenation, pulmonary artery pressure, pulmonary capillary wedge pressure, or pulmonary artery occlusion pressure, mixed venous oxygen content or saturation, arterial oxygen content or saturation, cardiac output, cardiac index, systemic vascular resistance, pulmonary vascular resistance, oxygen delivery, and oxygen extraction), chest radiograph and other radiological criteria, and standard physical examination of the patient. Determination of therapeutically effective amounts is well within the capability of those skilled in the art.

The decision to begin the therapy for a *Y. pestis* infection, particularly primary pneumonic plague, described herein may be based upon the appearance of the clinical manifestations of *Y. pestis* infection and appropriate laboratory testing for the presence of *Y. pestis* in the blood, lymph, or sputum. Typical clinical manifestations, particularly of pneumonic plague, are described herein above and include fever, chills, headache, fever, rapid or difficult breathing, pneumonia (particularly rapidly progressing pneumonia), blood in the sputum, bright red sputum, foamy red sputum, and rapid shock. *Y. pestis,* particularly the endotoxin associated with this Gram-negative bacterium, can lead to rapid septicemia (also referred to herein as "endotoxemia") and damage to tissues and organs, particularly in the lungs, where endotoxin or infiltration of the bacteria, or both may result in ALI. Alternatively, a physician may choose to initiate preventative methods for a patient at risk of developing pneumonic plague or, more generally, any *Y. pestis* infection, prior to the appearance of clinical symptoms. In the case of a bioterrorist attack, prophylactic measures may be deemed appropriate by medical professionals to minimize spread of the disease, tissue injury, shock, or death.

A physician of ordinary skill in the art can determine when treatment for *Y. pestis* infection (e.g., bubonic plague, septicemic plague, and pneumonic plague) should be initiated and for how long the treatment should continue. Such treatment decisions may be supported by standard clinical laboratory results which monitor the clinical manifestations of *Y. pestis* infection, particularly those of pneumonic plague. The methods of the invention may be practiced by continuously or intermittently administering a therapeutically effective dose of the A₁ adenosine receptor antagonist, alone or in combination with at least one other therapeutic agent (e.g., an antibiotic agent), for as long as deemed efficacious for the treatment of the *Y. pestis* infection. The decision to end therapy by the method of the invention may also be supported by standard clinical laboratory results indicating the disappearance of at least one of the clinical symptoms characteristic of a *Y. pestis* infection, particularly pneumonic plague. The therapy described herein may be restarted upon the return of the *Y. pestis* infection or a symptom thereof.

When a combination treatment is used, the combination of the A₁ adenosine receptor antagonist and the antibiotic agent or other therapeutic agent is administered at a concentration that is therapeutically effective to treat a *Y. pestis* infection, more particularly pneumonic plague. To accomplish this goal, the agents may be formulated using a variety of acceptable excipients known in the art. A₁ adenosine receptor antagonists and antibiotic agents may be administered, for example, by injection, either intravenously, intraperitoneally, intramuscularly, or subcutaneously. Methods to accomplish this administration are known to those of ordinary skill in the art. It may also be possible to obtain compositions which may be topically or orally administered, or which may be capable of transmission across mucous membranes.

The amount of a combination of at least one A₁ adenosine receptor antagonist and at least one other therapeutic agent (e.g., an antibiotic agent) to be administered is readily determined by one of ordinary skill in the art without undue experimentation. Factors influencing the mode of administration and the respective amount of the combination of agents disclosed herein include, but are not limited to, the severity of the disease, the history of the disease, and the age, height, weight, health, medical history (e.g., existence of other diseases such as diabetes, kidney or liver disease, and other drugs or treatments the patient is currently taking or has taken in the past), and physical condition of the individual undergoing therapy. Similarly, the amount of the combination of therapeutic agents disclosed herein to be administered will be dependent upon the mode of administration and whether the subject will undergo a single dose or multiple doses of these anti-plague agents. Generally, a higher dosage is preferred with increasing weight of the patient undergoing therapy.

The treatment of *Y. pestis* infection, particularly pneumonic plague, described herein can be accomplished with varying doses as well as dosage regimens. Treatment regimens will be based on doses and dosing schedules that maximize therapeutic effects. For example, the therapeutically effective amount of a combination of an A₁ adenosine receptor antagonist and an antibiotic agent can be readily determined by one of ordinary skill in the art without undue experimentation. In particular embodiments, the therapeutically effective dose of a combination of an A₁ adenosine receptor antagonist and an antibiotic may comprise doses of the individual agents that, when administered alone, would not be therapeutically effective or would be less therapeutically effective than when administered in combination with each other. Thus, when an A₁ adenosine receptor antagonist of the invention and an antibiotic agent are administered in combination, a synergistic therapeutic effect may be observed. "Synergistic therapeutic effect" refers to a therapeutic effect observed with a combination of two or more therapies (in this case, the A₁ adenosine receptor antagonist and the antibiotic agent) wherein the therapeutic effect (as measured by any of a number of parameters) is greater than the sum of the respective individual therapeutic effects observed with the respective individual therapies. The combination of an A₁ adenosine receptor antagonist and at least one additional therapeutic agent (e.g., an antibiotic agent) may produce a synergistic effect that permits a reduction in the dosages of these agents and an improvement of the clinical outcome of the subject being treated. A reduced dose of the A₁ adenosine receptor antagonist and the additional therapeutic agent(s) may in turn reduce unwanted side effects associated with each agent.

In some embodiments of the invention, the method comprises administration of multiple doses of an A₁ adenosine receptor antagonist in combination with multiple doses of a second therapeutic agent, such as, for example, an antibiotic agent. The method may comprise administration of 1, 2, 3, 4, 5, 6, 7, 8, 9, 10, 15, 20, 25, 30, 35, 40, or more therapeutically effective doses of a pharmaceutical composition comprising either an A₁ adenosine receptor antagonist or an antibiotic agent, or both. The frequency and duration of administration of multiple doses of the pharmaceutical compositions can be readily determined by one of skill in the art without undue experimentation. Moreover, treatment of a subject with a therapeutically effective amount of a combination of an A₁ adenosine receptor antagonist and, for example, an antibiotic agent can include a single treatment or can include a series of treatments. It will also be appreciated that the effective dosage of an A₁ adenosine receptor antagonist or an antibiotic agent used for treatment may increase or decrease over the course of a particular treatment. Changes in dosage may result and become apparent from the results of diagnostic assays known in the art.

Many of the A₁ adenosine receptor antagonist compounds of the present invention can be provided as solvates, hydrates, and salts with pharmaceutically compatible counterions. Such pharmaceutically acceptable base addition salts are those salts that retain the biological effectiveness and properties of the free acids, and that are obtained by reaction with suitable inorganic or organic bases. The A₁ adenosine receptor antagonists of the invention may form pharmaceutically acceptable salts with both organic and inorganic acids and bases. Exemplary weak organic acids for salt formation include but are not limited to acetic acid, beta-alanine, d1-alanine, D-alanine, L-alanine, formic acid, propanoic acid, butyric acid, palmetic acid, oleic acid, sebacic acid, cinnamic acid, adipic acid, citric acid, ascorbic acid (vitamin C), lactic acid, malic acid, maleic acid, fumaric acid, tartartic acid, dl-glutamic acid, D-glutamic acid, L-glutamic acid, dl-aspartic acid, D-aspartic acid, L-aspartic acid, glycine, succinic acid, glutaric acid, gluconic acid, benzoic acid, p-chlorobenzoic acid, p-hydroxybenzoic acid, p-methoxybenzoic acid, o-hydroxybenzoic acid (salicylic acid), 1-hydroxy-2-naphthoic acid, 3-hydroxy-2-naphthoic acid, and the like. Strong organic acids that may be used for salt formation include, for example, benzenesulfonic acid, p-toluenesulfonic acid, m-nitrobenzenesulfonic acid, methanesulfonic acid, ethanesulfonic acid, 1-naphthalenesulfonic acid, 2-naphthalenesulfonic acid, laurylsulfonic acid, and the like. Examples of strong inorganic acids for salt formation include hydrochloric acid, hydrobromic acid, hydroiodic acid, sulfuric acid, phosphoric acid, nitric acid, sodium bisulfate, potassium bisulfate, sodium hydrogen phosphate, potassium hydrogen phosphate, boric acid, xinafoic acid(i.e., xinafoate salt is formed with 1-hydroxy-2-naphthoic acid) and the like.

A xinafoate salt (1-hydoxy-2-napthoic acid) of an A₁ adenosine receptor antagonist may be administered in combination with, for example, an antibiotic agent to treat or prevent tissue injury and sepsis associated with a *Y. pestis* infection, particularly pneumonic plague, in a subject. Xinafoate salts, such as salmeterol xinafoate, are known and have been synthesized in the art. See, for example, *Merck Index*, *supra*, and U.S. Patent No. 4,992,474, both of which are herein incorporated by reference in their entirety. Because xinafoate salts are known to be largely insoluble and to exhibit reduced oral absorption, such salts may be particularly potent, safe, and efficacious when administered by pulmonary inhalation. Inhalational therapy with a xinafoate salt of an A₁ adenosine receptor antagonist of the invention may minimize negative systemic effects associated with the traditional A₁ adenosine receptor antagonist agents. Inhalation of the A₁ adenosine receptor antagonists of the invention as xinafoate salts may permit more direct contact with the therapeutic agent and the lung, for example, in the case of primary or secondary pneumonic plague, wherein the bacteria colonize and release endotoxin in the lungs.

The A₁ adenosine receptor antagonist, whether provided alone or in combination with at least one other therapeutic agent such as an antibiotic, is typically provided by standard techniques within a pharmaceutically acceptable buffer; for example, sterile saline, sterile buffered water, propylene glycol, combinations of the foregoing, etc. The A₁ adenosine receptor antagonist and additional therapeutic agent (e.g., an antibiotic agent), can be formulated in separate pharmaceutical compositions, or can be formulated within a single pharmaceutical composition for simultaneous administration. Methods for preparing parenterally administrable agents are described in Remington's Pharmaceutical Sciences (18th ed.; Mack Publishing Company, Eaton, Pennsylvania, 1990), herein incorporated by reference.

The A₁ adenosine receptor antagonists of the invention and other therapeutic agents of the invention (e.g., an antibiotic agent), can be administered alone, but may also be administered in admixture with a pharmaceutically acceptable carrier selected with regard to the intended route of administration and pharmaceutical practice. Thus, a pharmaceutical composition of the invention is formulated to be compatible with its intended route of administration. Examples of possible routes of administration include parenteral, (e.g., intravenous (IV), intramuscular (IM), intradermal, subcutaneous (SC), intraperitoneal (IP), or infusion), oral, pulmonary (e.g., inhalation), nasal, transdermal (topical), transmucosal, and rectal administration. A "pharmaceutically acceptable carrier" refers to a carrier that is conventionally used in the art to facilitate the storage, administration, or the therapeutic effect of the active ingredient. A suitable carrier may also reduce any undesirable side effects of the A₁ adenosine receptor antagonist or the at least one additional therapeutic agent (e.g., an antibiotic agent), in the case of combination therapy. It should not produce significant local or systemic adverse effects in recipients at the dosages and concentrations employed for treatment. Pharmaceutically acceptable carriers of the invention may further comprise surfactants, such as those disclosed in U.S. Patent Nos. 6,652,837 and 6,613,307, which are herein incorporated by reference in their entirety. Methods for formulating pharmaceutical compositions are generally known in the art. A thorough discussion of formulation and selection of pharmaceutical acceptable carriers, stabilizers, and isomolytes can be found in Remington's Pharmaceutical Sciences (18th ed.; Mack Publishing Company, Eaton, Pennsylvania, 1990), herein incorporated by reference.

When a composition of the invention is administered by intravenous, intradermal, or subcutaneous injection, the composition is in the form of a pyrogen-free, parenterally acceptable aqueous solution. The preparation of such parenterally acceptable solutions, having due regard to pH, isotonicity, stability, and the like, is well within the skill in the art. Solutions or suspensions used for parenteral, intradermal, subcutaneous, or intravenous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, multiple dose vials made of glass or plastic, or plastic bags of intravenous solutions, e.g. dextrose, ringers lactate or normal saline.

The A₁ adenosine receptor antagonists of the invention can be formulated for parenteral administration by injection, e.g., by bolus injection or continuous infusion. Formulations for injection can be presented in unit dosage form, e.g., in ampoules or in multidose containers, with an added preservative. The compositions can take such forms as suspensions, solutions, or emulsions in oily or aqueous vehicles, and can contain formulatory agents such as suspending, stabilizing, and/or dispersing agents.

For oral administration, the A₁ adenosine receptor antagonists can be formulated by combining a compound of formula (I) with pharmaceutically acceptable carriers well known in the art. Such carriers enable the present compounds to be formulated as tablets, pills, dragees, capsules, liquids, gels, syrups, slurries, suspensions and the like, for oral ingestion by a patient to be treated. Pharmaceutical preparations for oral use can be obtained by adding a compound of formula (I) with a solid excipient, optionally grinding a resulting mixture, and processing the mixture of granules, after adding suitable auxiliaries, if desired, to obtain tablets or dragee cores. Suitable excipients include, for example, fillers and cellulose preparations. If desired, disintegrating agents can be added.

For administration by inhalation, A₁ adenosine receptor antagonists of the present invention are conveniently delivered in the form of an aerosol spray presentation from pressurized packs or a nebulizer, with the use of a suitable propellant. In the case of a pressurized aerosol, the dosage unit can be determined by providing a valve to deliver a metered amount. Capsules and cartridges of, e.g., gelatin, for use in an inhaler or insufflator can be formulated containing a powder mix of the compound and a suitable powder base such as lactose or starch. Methods and devices for administering compositions via pulmonary inhalation and for producing particles suitable for such administration are disclosed in the art. See, for example, U.S. Patent Nos. 6,221,338, 6,475,523, 6,521,260, 6,582,678, 6,941,948, 6,948,496, 6,989,155; U.S. Patent Application Publication Nos. 2003/0170183, 2003/0202944, 2005/0013862, 2005/0152849, 2005/0158394, 2005/0205083, and 2006/0029552; all of which are herein incorporated by reference in their entirety.

The exact formulation, route of administration, and dosage of the A₁ adenosine receptor antagonist and antibiotic agent can chosen by the individual physician in view of the patient's condition. Dosage amount and dosing intervals can be adjusted individually to provide plasma levels of the A₁ adenosine receptor antagonist and antibiotic agent that are sufficient to maintain positive therapeutic effects.

One of skill in the art will appreciate that the methods for preventing and treating *Y. pestis* infections such as bubonic plague, septicemic plague, and pneumonic plague disclosed herein can be combined with any other therapy for a *Y. pestis* infection. Such therapies include but are not limited to fluid therapy, vasopressor therapy, blood product administration, steroids, insulin, anti-sepsis agents, such as recombinant activated protein C (APC; drotrecogin alpha; Xigris^{®}) or anti-endotoxin therapies, circulatory support, ventilatory support, or renal support. These additional therapies may be of particular relevance to pneumonic plague. The skilled artisan will further recognize that the disclosed methods for preventing and treating tissue injury and sepsis associated with *Y. pestis* infections, particularly pneumonic plague, more particularly primary pneumonic plague, may be combined with any disease containment technique known in the art (e.g., isolation of patients suffering from a *Y. pestis* infection, appropriate protection of medical staff treating patients suffering from *Y. pestis* infections, etc.).

The present invention also provides for the use of an A₁ adenosine receptor antagonist in the manufacture of a medicament for treating a subject for a *Y. pestis* infection, particularly pneumonic plague. In particular embodiments, the medicament is coordinated with treatment using at least one additional therapeutic agent such as an antibiotic agent. By "coordinated" is intended that the medicament comprising the A₁ adenosine receptor antagonist is to be used either prior to, during, or after treatment of the subject using, for example, an antibiotic agent. "Treatment" in the context of coordinated use of a medicament comprising an A₁ adenosine receptor antagonist described herein with one or more therapeutic agents, particularly antibiotic agents, is herein defined as the application or administration of the medicament to a subject, where the subject has a *Y. pestis* infection, a symptom associated with a *Y. pestis* infection, or a predisposition toward development of a *Y. pestis* infection, more particularly pneumonic plague, where the purpose is to cure, heal, alleviate, relieve, alter, remedy, ameliorate, improve, or affect the condition, any associated symptoms of *Y. pestis* infection, or the predisposition toward the development of a *Y. pestis* infection.

The present invention also provides for the use of a synergistic combination of an A₁ adenosine receptor antagonist in the manufacture of a medicament for treating a subject for a *Y. pestis* infection, particularly pneumonic plague, more particularly primary pneumonic plague, wherein the medicament is coordinated with treatment using at least one additional therapeutic agent, including but not limited to an antibiotic agent. By "synergistic combination" is intended that the medicament comprising an amount of the A₁ adenosine receptor antagonist provides for a synergistic therapeutic effect when the medicament is coordinated with treatment using an additional therapeutic agent such as an antibiotic agent in the manner set forth herein above. "Synergistic therapeutic effect" refers to a therapeutic effect observed with a combination of two or more therapies (in this case, the A₁ adenosine receptor antagonist and, for example, the antibiotic agent) wherein the therapeutic effect (as measured by any of a number of parameters) is greater than the sum of the respective individuals therapeutic effects observed with the respective individual therapies.

The article "a" and "an" are used herein to refer to one or more than one (i.e., to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation.

### EXPERIMENTAL

### Example 1: Evaluation of L-97-1 Alone or in Combination with Antibiotics in the Treatment of Y. Pestis Infection

### Y. pestis strain to be evaluated

Evaluation of L-97-1 in the treatment of pneumonic plague will be evaluated using *Y*. *pestis* CO99*pgm-*, an attenuated strain derived from the 1992 *orientalis* pandemic organism that expresses *lcr*+-dependent plasmid-derived Yop proteins and a full complement of endotoxin-related lipopolysaccharide (LPS). This strain lacks chromosomal virulence factors associated with the pigmentation locus.

### Infection of Rats with Y. pestis

Rats will be infected by intratracheal injection of *Y. pestis* and then treated with antibiotics with or without L-97-1. Post-infection treatment will be initiated at 12 hours or 24 hours. The effects of L-97-1 alone *vs.* L-97-1 plus antibiotics on the following primary endpoints will be assessed: 1) 5 day mortality and 2) existence of ALI, as determined by lung histopathology, neutrophil influx into alveolar septa and airspaces, and wet/dry weight (W/D) ratio at 24 and 72 hours post-infection with *Y. pestis.* In addition, bacterial dissemination and seeding to distal organs, quantitative blood cultures, and plasma levels of endotoxin and L-97-1 will be determined in selected treatment subgroups.

### Surgical Procedures

Male pathogen-free Sprague-Dawley rats (275-325 g, Harlan, Indianapolis, Indiana) are housed in positive-pressure isolation carrels with free access to food and water before experiments and in BSL-2 biohazard laboratory or animal care suites after infection. Under general anesthesia (3% isoflurane, 97% O₂) and in accordance with aseptic technique throughout, the left carotid artery and right jugular vein are catheterized (PE-50; Clay Adams, Parsippany NJ). Baseline vital signs (rectal temperature, blood pressure, pulse rate, and respiratory frequency) are determined, and an initial arterial blood sample is obtained (1.2 ml) for hematology, blood gases, and culture, as described herein below. Withdrawn blood is replaced with 3x the volume of sterile 0.9% NaCl (normal saline; "NS"). Animals are infected intratracheally as described below, and the surgical incision closed. Anesthesia is withdrawn, and the animal is monitored until it regains consciousness (typically 3-5 minutes) and is then returned to its home cage. Catheters are shielded by plastic sleeves and suspended by counterweights to allow free movement and access to food and water.

### Bacterial cultures

*Y*. *pestis* (CO99*pgm-*) is streaked on HIB plates and cultured for 72 hours at 25°C. Fresh HIB broth is inoculated with 5-10 isolated colonies and incubated for 8 hours at 25°C, 100 rpm. Fresh HIB broth (25 mL) is inoculated with 25-50 µL of 8 hour cultures and incubated for 18-24 hours at 25°C, 100 rpm. Log-phase organisms are collected from the 18-24 hour suspensions, sedimented (1000 x g, 10 min, 4°C), washed twice with NS, resuspended in NS to the desired inoculum in 0.1 mL total volume per rat, and held at 4°C until use. Inocula are enumerated using a hemacytometer and verified by replicate streak culture on nutrient agar (24 hours, 37°C) to determine the total number of colony-forming units (cfu) actually administered to each animal. Whole blood (10-20 µL) and organ homogenates from infected animals, as described below, are likewise serially diluted and streak-plated on nutrient agar for culture quantitation. See Lechner et al. (1993) Micro. Res. Tech. 26:444-456; Matuschak et al. (1994) Am. J. Respir. Crit. Care Med. 149:41-49; Lechner et al. (1997) J. Crit. Care 12:28-38; Lechner et al. (1998) Am. J. Respir. Crit. Care Med. 157:1550-1558; Lechner et al. (2004) Am. J. Respir. Crit. Care Med. 169:A232; Matuschak et al. (2004) Crit. Care Med. 32:A479; Mallea et al. (2003) Am. J. Respir. Crit. Care Med. 167:A859; Matuschak et al. (2001) Am. J. Respir. Crit. Care Med. 163:1002-1009; Lechner et al. (1992) Am. J. Physiol. 263:L526-L535; Lechner et al. (1994) Am. J. Physiol. 266:L561-L568; Lechner and Matuschak (1998) Intens. Med. Emerg. Med. 35:106-113; Macarthur et al. (2003) Crit. Care Med. 31:237-245; and Knuepfer et al. (2004) Crit. Care Med. 32:175-183.

### Intratracheal infection with Y. pestis

Following catheterization and determination of baseline indices, rats are infected with graded inocula of C099pgm- by intratracheal injection over 15-20 sec (normally 5-6 breaths). See Lechner et al. (2004) Am. J. Respir. Crit. Care Med. 169:A232; Matuschak et al. (2004) Crit. Care Med. 32:A479; and Mallea et al. (2003) Am. J. Respir. Crit. Care Med. 167:A859. Each inoculum is suspended in a total volume of 100 µL NS and injected via sterile 15 mm, 25-gauge needle directed caudally. The animal is gently rotated into the left and right decubitus positions once during infection to facilitate dispersal of bacteria into both lungs. Based upon preliminary studies, three doses of C099pgm- will be used, ranging from 1 x 10⁷ to 1 x 10⁹ cfu/animal (Groups 2-4) to define a dose that reproducibly initiates ALI, disseminated infection to key systemic organs (e.g., the liver and spleen), and death over 3-5 days in the absence of intervention. The determined dose will be used subsequently to infect treatment groups 6-18. Vital signs are recorded and additional arterial bleeds are obtained at specified hourly or daily intervals until sacrifice and necropsy. Phlebotomies for any animal are limited to four per 24 hour period.

### Post-infection treatment regimens

Infected rats receive either NS vehicle or a graded dose of L-97-1 over 8 hours/day as a continuous intravenous (i.v.) infusion via the indwelling jugular catheter. The intervals between infection with C099pgm- and initiation of treatments are summarized in Table 1. Such treatments are continued for up to 72 hours post-infection or until a scheduled necropsy. Similarly, doses of antibiotic (e.g., ciprofloxacin) are administered twice daily (b.i.d) commencing at 12 or 24 hours after infection via the jugular catheter until sacrifice. The antibiotic administrations do not exceed 72 hours thereafter.

**Table 1: Treatment Protocol**

| Group | Injury | Dose of *Y*. *pestis* | Initiation of Treatment | L-97-1 | Antibiotic |
|---|---|---|---|---|---|
| 1 | No | - | No | - | - |
| 2 | Yes | 1 x 10⁷ CFU | No | - | - |
| 3 | Yes | 1 x 10⁸ CFU | No | - | - |
| 4 | Yes | 1 x 10⁹ CFU | No | - | - |
| 5 | Yes | 1 x 10⁹ CFU | 12 hour | 5 mg/kg/h | - |
| 6 | Yes | 1 x 10⁹ CFU | 12 hour | 10 mg/kg/h | - |
| 7 | Yes | 1 x 10⁹ CFU | 12 hour | 20 mg/kg/h | - |
| 8 | Yes | 1 x 10⁹ CFU | 12 hour | - | Ciprofloxacin (10 mg/kg i.v., *b.i.d.)* |
| 9 | Yes | 1 x 10⁹ CFU | 12 hour | 5 mg/kg/h | Ciprofloxacin (10 mg/kg i.v., *b*.*i*.*d*.) |
| 10 | Yes | 1 x 10⁹ CFU | 12 hour | 10 mg/kg/h | Ciprofloxacin (10 mg/kg i.v., *b.i.d.)* |
| 11 | Yes | 1 x 10⁹ CFU | 12 hour | 20 mg/kg/h | Ciprofloxacin (10 mg/kg i.v., *b*.*i*.*d*.) |
| 12 | Yes | 1 x 10⁹ CFU | 24 hour | 5 mg/kg/h | - |
| 13 | Yes | 1 x 10⁹ CFU | 24 hour | 10 mg/kg/h | - |
| 14 | Yes | 1 x 10⁹ CFU | 24 hour | 20 mg/kg/h | - |
| 15 | Yes | 1 x 10⁹ CFU | 24 hour | - | Ciprofloxacin (10 mg/kg i.v., *b.i.d.)* |
| 16 | Yes | 1 x 10⁹ CFU | 24 hour | 5 mg/kg/h | Ciprofloxacin (10 mg/kg i.v., *b.i.d.)* |
| 17 | Yes | 1 x 10⁹ CFU | 24 hour | 10 mg/kg/h | Ciprofloxacin (10 mg/kg i.v., *b.i.d.)* |
| 18 | Yes | 1 x 10⁹ CFU | 24 hour | 20 mg/kg/h | Ciprofloxacin (10 mg/kg i.v., *b.i.d.)* |

30 rats will be evaluated per treatment group, owing to 10 rats per time point x 3 time points, and 18 primary groups = 540 rats total.

### Administration of L-97-1 and ciprofloxacin

Treatment is initiated at either 12 or 24 hours post-infection. L-97-1 is administered as a continuous i.v. infusion for 8 hours/day for up to 72 hours, depending upon time of sacrifice and necropsy. Based on pharmacokinetic data following i.v. administration, the plasma half life of L-97-1 in rats is 30-60 minutes. L-97-1 will be given as an i.v treatment to obtain adequate plasma levels. Ciprofloxacin is administered b.i.d. by intravenous injection for up to 72 hours, depending upon time of sacrifice and necropsy, as set forth in Table 1.

### Necropsies and postmortem studies

Rats are sacrificed at 24 hours, 72 hours, or 5 days after infection by deep isoflurane anesthesia, using the same induction chamber employed to prepare animals for surgery (1-2 minutes elapsed time). See Lechner et al. (1993) Micro. Res. Tech. 26:444-456; Matuschak et al. (1994) Am. J. Respir. Crit. Care Med. 149:41-49; Lechner et al. (1997) J. Crit. Care 12:28-38; Lechner et al. (1998) Am. J. Respir. Crit. Care Med. 157:1550-1558; Lechner et al. (2004) Am. J. Respir. Crit. Care Med. 169:A232; Matuschak et al. (2004) Crit. Care Med. 32:A479; Mallea et al. (2003) Am. J. Respir. Crit. Care Med. 167:A859; Matuschak et al. (2001) Am. J. Respir. Crit. Care Med. 163:1002-1009; Lechner et al. (1992) Am. J. Physiol. 263:L526-L535; Lechner et al. (1994) Am. J. Physiol. 266:L561-L568; Lechner and Matuschak (1998) Intens. Med. Emerg. Med. 35:106-113; Macarthur et al. (2003) Crit. Care Med. 31:237-245; and Knuepfer et al. (2004) Crit. Care Med. 32:175-183. Aseptic laparotomy is then rapidly performed to facilitate exsanguination of each rat via the abdominal aorta to collect a final large arterial bleed (at least 3-5 mL). This final bleed is followed by penetration of the diaphragm immediately beneath the xiphoid process to create an observed pneumothorax.

The thoracic cavity is opened and the right mainstream bronchus is ligated in *situ,* and the trachea is cannulated by tracheotomy. Right lung lobes are excised to determine the wet/dry ratio as an estimate of microvascular permeability by drying at 70°C to constant weight and content of CO99pgm- by homogenization and quantitative culture. The entire left lung is fixed *in situ* with cacodylate-buffered 2% gluteraldehyde at a transpulmonary inflation pressure of 20-22 cm H₂O for 30 minutes and then 2-3 mm midlobar slices are immersed in gluteraldehyde overnight at 5°C. Paraffin-embedded serial sections (6 µm) are stained with either hematoxylin plus eosin for routine evaluation of histopathology, or with chloroacetate esterase plus hematoxylin to visualize neutrophils. See Lechner et al. (1993) Micro. Res. Tech. 26:444-456; Matuschak et al. (2004) Crit. Care Med. 32:A479; and Matuschak et al. (2001) Am. J. Respir. Crit. Care Med. 163:1002-1009. Consistent segments of the central hepatic lobe and the spleen are isolated for wet/dry determinations, homogenization for quantitative culture, and for immersion fixation, sectioning, and staining as for the left lung. Rats dying overnight are recorded as having survived to the previous observation period but are not necropsied.

Using a double-blind system, the following parameters are used to evaluate lung sections for *Y. pestis* ALI: (1) presence or absence of interstitial or alveolar edema, (2) presence or absence of interstitial or alveolar hemorrhage, and (3) total number of neutrophils per high power field. A minimum of 5 fields (400x magnification) are evaluated for at least 5 animals. Regions showing artifactual damage such as crushing of the pleura or compression-induced atelectasis are excluded from analysis.

### Statistical analysis

Microsoft Excel and Access programs are used to store results. Mean and standard errors are determined for each control (or sham) or experimental group consisting of 6 or more animals. Significance of variance within the group and differences between groups are analyzed using one-way or multivariate ANOVA following with Bonferroni correction and/or Newman-Keuls tests. Assessments of significance of relationships between dependent (measured) and independent variables, when required, is made using regression analyses and determinations of correlation coefficients. Null hypothesis, for intra-group variance/inter-group difference(s), or relationships between dependent and independent variables, is rejected at P values less than 0.05. The seven day mortality data are plotted as survival curves using GraphPad Prism, version 4.01 (GraphPad Software, Inc., San Diego, CA). For the seven day mortality studies, the survival curves for each group are compared to either *Y. pestis* control or antibiotics alone using the Mantel-Haenszel logrank test to determine the one-tail P-value. The survival curves are analyzed using the logrank test for trend. The level of significance is set at P < 0.05. Measurements for histopathology, #PMNs/HPF and scores for presence of edema and hemorrhage are analyzed with the use of the Student's *t* test for unpaired data. The level of significance is set at P < 0.05

### Example 2: Evaluation of L-97-1 Alone or in Combination with Antibiotics on Survival Rates and Lung Injury Following Y. Pestis Infection

Following NIH Guidelines for the Care and Use of Animals male pathogen-free Sprague-Dawley rats (275-325 g, Harlan, Indianapolis) were housed in positive-pressure isolation carrels with free access to food and water before experiments and in biosafety level 2 (BSL-2) biohazard laboratory or animal care suites thereafter. Under general anesthesia (3% isoflurane, 97% O₂) and using aseptic technique throughout, the left carotid artery and right jugular vein were catheterized (PE-50; Clay Adams, Parsippany NJ). Catheters were shielded by stainless steel sleeves and swivels to allow free movement and access to food and water.

Following catheterization and determination of baseline indices, rats were infected with enumerated inocula of *Y. pestis,* C099pgm- by intratracheal (i.t.) injection, 1 x 10⁸ cfu/animal over 15-20 sec (normally 5-6 breaths). Importantly, this strain of *Y. pestis* was derived directly from the human clinical isolate CO92 (an *occidentalis* biotype that caused fatal pneumonic plague in 1992) and retains all chromosomal and plasmid-derived virulence factors of wild-type plague, including a type III secretion system or "injectisome" composed of *Yersinia* outer proteins (Yops), while lacking only the pigmentation locus that encodes an iron-transport siderophore. Each inoculum was suspended in a total volume of 100 µL NS and injected via a sterile 15 mm 25-gauge needle directed caudally. The animal was gently rotated into the left and right decubitus positions once during infection to facilitate dispersal of bacteria into both lungs.

Infected rats received either an effective antibiotic (ciprofloxacin, 10 mg/kg) administered at specified times after infection *b*.*i*.*d* plus a continuous infusion of pharmacy grade sterile water (H2O) (0.5 ml/h) or ciprofloxacin plus L-97-1 (1, 5, 10, or 20 mg/kg/h) as a continuous intravenous (i.v.) infusion over 8 h/day via the indwelling jugular venous catheter. Such treatments of ciprofloxacin (cipro) plus H2O or of cipro plus L-97-1 began 72 h after i.t. infection and continued for up to three full days (72 h) of therapy post-infection. In previous experiments the 6-day survival rate for animals receiving ciprofloxacin starting 24 h after infection was found to be 100% (10/10), versus 30% (3/10) in animals given an infusion of H2O alone starting at 24 h post infection and versus 33% (5/15) in animals with no intervention (*Y. pestis* infection controls).

### Determination of acute lung injury (ALI) scores with histopathology

Animals that survived to 6 days (144 h) were necropsied, while rats dying overnight were recorded as having survived to the previous observation period but were not necropsied. Following aseptic laparotomy and exanguination of each rat via the abdominal aorta, penetration of the diaphragm immediately beneath the xiphoid process was performed to create an observed pneumothorax. The thoracic cavity was opened and the entire left lung was fixed *in situ* with phosphate-buffered 10% formaldehyde at a transpulmonary inflation pressure of 20-22 cm H₂O for 30 min and then 2-3 mm thick midsagittal slices of the entire left lung were immersed in fresh buffered formaldehyde overnight at 5°C. Paraffin-embedded serial sections (6 µm) of each sagittal lung slice were stained with hematoxylin plus eosin (H&E) for routine evaluation of histopathology.

Using a double-blind scoring system, these H&E-stained sections were evaluated at magnifications of 40X, 100X and 400X on a scale of 0 (normal), 1 (mild), 2 (moderate), or 3 (severe) for parenchymal injuries of the following types: (a) interstitial and/or alveolar edema; (b) interstitial and/or alveolar hemorrhage; and (c) interstitial and alveolar leukocytic infiltration indices (LII). For the first two types of such injuries, the entire sagittal section was scanned at these multiple magnifications to assign a global severity index to each lung. In practice, at least ten random high powered fields (HPV) were assessed per sagittal section, starting at very low magnification (40X) and then magnifying these fields up to 400X as needed to denote a specific injury type such as interstitial or alveolar edema or hemorrhage. For LII, a total of eleven HPF for each lung section at 400X were independently selected by microscope stage coordinates and the median LII score of these 11 HPFs was reported. Regions showing artifactual damage such as crushing of the pleura or compression-induced atelectasis were excluded from analysis.

### Statistical analysis

Survival curves made in GraphPad Prism v. 4.01 (GraphPad Software, Inc., San Diego, CA) were compared between groups using the Mantel-Haenszel logrank test to determine the one-tail P-value. Lung scores determined by histopathology were analyzed with the use of the Student's *t* test for unpaired data. The level of significance in all cases was set at P < 0.05. With respect to assuring the adequacy of sample sizes to achieve statistical significance among groups, group sizes of n = 10 rats each provided the power to detect a 20% difference in the proposed criteria at the *P* < 0.05 level when analyzed using a repeated measures model for split-plot design (univariate mixed ANOVA).

### Results - Survival times and Survival Rates

At 72 h post-*Y. pestis* infection, L-97-1 plus ciprofloxacin (cipro) at 1 mg/kg/h, 5 mg/kg/h, 10 mg/kg/h or 20 mg/kg/h improved the median survival time from 96 h and 84 h in the no intervention and cipro alone test groups, respectively, to greater than 144 h for the L-97-1 plus cipro groups. Percent survival rates also increased from 28% and 33% for the cipro alone and no intervention test groups, respectively to 60-70 % in a dose-dependent fashion for the L-97-1 plus cipro group (p = 0.02, Logrank test). These results are summarized in Table 2 and Figure 1.

**Table 2: Survival Summary Data**

| Intratracheal (i.t.) infections with 1x10⁸ cfu *Y. pestis* CO99*pgm*- All interventions *t* = 72 h after i.t. infection | | |
|---|---|---|
| *Group* | *Median Survival Time* | *Survival* |
| No Intervention (n = 15) | 96 h | 33% |
| Cipro (10 mg/kg b.i.d) + H2O (*n* = 18) | 84 h | 28% |
| Cipro + L-97-1 (1 mg/kg/h) (*n* = 10) | > 144 h | 60% |
| Cipro + L-97-1 (5 mg/kg/h) (*n* = 10) | > 144 h | 70% |
| Cipro + L-97-1 (10 mg/kg/h) (*n* = 10) | > 144 h | 70% |
| Cipro + L-97-1 (20 mg/kg/h) (*n* = 13) | > 144 h | 69% |

### Results - Lung Injury Measurements

At 72 h post-*Y. pestis* infection, ciprofloxacin (cipro) alone did not significantly improve lung edema, leukocyte infiltration index (LII), or lung scores (i.e., combined interstitial and alveolar edema and hemorrhage and LII scores) versus no intervention. The administration of L-97-1 in combination with cipro significantly improved lung edema versus no intervention or cipro alone, LII versus no intervention, and lung scores versus no intervention or cipro alone. Lung edema results, LII scores, and lung scores are represented graphically in Figures 2, 3, and 4, respectively.

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. An A₁ adenosine receptor antagonist for use in treating or preventing tissue injury and sepsis associated with a *Yersinia pestis* (*Y. pestis)* infection in a subject, wherein the A₁ adenosine receptor antagonist comprises a compound of formula (I): 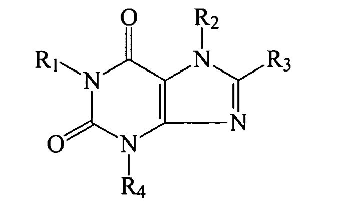 wherein R₁ is C₁-C₈ alkyl;
R₂ is of the formula: 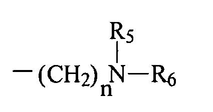 wherein n is an integer of from 1 to 8; R₅ is H or CH₃(CH₂)ₚ, wherein p is an integer of from 1 to 7; and R₆ is H; (CH₂)ₘH; or (CH₂)ₘOH, wherein m is an integer of from 1 to 8;
R₃ is:
-(CH₂)_{q}C₆H₄-R₇
wherein q is an integer of from 1 to 8; wherein R₇ is selected from H, OH, NH₂, R₉COOH, wherein R₉ is an alkylene or alkenylene group having from 1 to 8 carbon atoms, and (CH₂)ₜOH, wherein t is an integer of from 1 to 8; and
R₄ is of the formula: 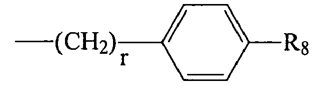 wherein R₈ is selected from H, NH₂, OH, (CH₂)_{f}NH₂, wherein f is an integer of from 1 to 8, (CH₂)ₛOH, wherein s is an integer of from 1 to 8, and R₁₀COOH, wherein R₁₀ is an alkylene or alkenylene group having from 1 to 8 carbon atoms; and r is an integer of from 1 to 8,
or a pharmaceutically acceptable salt, hydrate or solvate thereof.

2. An A₁ adenosine receptor antagonist as claimed in claim 1 wherein:
R₁ is C₃ alkyl;
R₂ is: 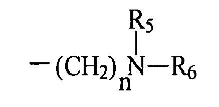 wherein n is 2; R₅ is CH₃(CH₂)ₚ, wherein p is 1; and R₆ is (CH₂)ₘOH, wherein m is 2;
R₃ is:
-(CH₂)_{q}C₆H₄-R₇
wherein q is 1; wherein R₇ is H; and
R₄ is of the formula: 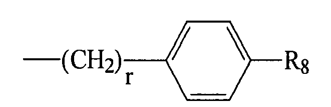 wherein R₈ is NH₂; and r is 2.

3. An A₁ adenosine receptor antagonist as claimed in claim 1 or 2 wherein the subject is a human patient.

4. An A₁ adenosine receptor antagonist as claimed in claimed in any one of claims 1 to 3 wherein the *Y. pestis* infection is selected from bubonic plague, septicemic plague, and pneumonic plague.

5. An A₁ adenosine receptor antagonist as claimed in any one of the preceding claims wherein the *Y. pestis* infection is primary or secondary pneumonic plague.

6. An A₁ adenosine receptor antagonist as claimed in any one of the preceding claims wherein said treating or preventing tissue injury and sepsis associated with a *Y. pestis* infection further comprises administration of an antibiotic agent.

7. An A₁ adenosine receptor antagonist as claimed in claim 6 wherein the antibiotic agent is selected from β-lactams, aminoglycosides, carbapenems, cephalosporins, penicillin, amoxicillin, clindamycin, carboxypenicillins, ureidopenicillins, β-lactamase inhibitors, fluoroquinones, glycopeptides, oxazolidinones, streptogramins, monobactams, and polymyxins.

8. An A₁ adenosine receptor antagonist as claimed in claim 6 or claim 7 wherein the antibiotic agent is gentamicin, tobramycin, clinamycin, cefotaxime, amikacin, imipenem, netilycin, ceftazidime, cefuroxamine, metronidazole, cefazolin, cefoperazone, ceftriaxone, mezlocilin, ampicillin, amoxiclav, piperacillin, tazobactam, ciprofloxacin, aztreonam, polymyxin B, or colistin.

9. An A₁ adenosine receptor antagonist as claimed in any one of claims 6 to 8 wherein the A₁ adenosine receptor antagonist and the antibiotic agent are administered sequentially or simultaneously.

10. An A₁ adenosine receptor antagonist as claimed in any one of the preceding claims wherein the A₁ adenosine receptor antagonist is administered intravenously, intramuscularly, intradermally, subcutaneously, orally, nasally, transdermally, transmucosally, rectally, intraperitoneally, by pulmonary administration, or by infusion.

11. An A₁ adenosine receptor antagonist as claimed in any one of the preceding claims which is for use in preventing or limiting organ injury associated with the *Y. pestis* infection.

12. An A₁ adenosine receptor antagonist as claimed in claim 11, wherein the organ is a lung.

13. An A₁ adenosine receptor antagonist as claimed in claim 12 wherein the damage to the lung constitutes acute lung injury (ALI).

14. An A₁ adenosine receptor antagonist as claimed in any one of the preceding claims which is for use in preventing or limiting the development of sepsis induced by the *Y. pestis* infection.

15. An A₁ adenosine receptor antagonist as claimed in any one of the preceding claims which is for use in preventing tissue injury and sepsis associated with a *Y. pestis* infection in a patient at risk of developing pneumonic plague or in a population of human patients exposed to *Y. pestis* during a bioterrorist attack.
